# EUROPEAN PATENT APPLICATION

(11) **EP 4 389 726 A1**
(43) Date of publication of application: **26.06.2024**
(21) Application number: 23210494.3
(22) Date of filing: 17.11.2023
(51) Int. Cl.: C07C 1/32, C07C 13/04, C07C 17/10, C07C 21/04, A01N 27/00

(54) **METHOD OF PREPARING 1-(2,2-DIMETHYLPROPYL)-CYCLOPROPENE AS SPRAYABLE ETHYLENE ANTAGONIST IN PLANTS AND USES THEREOF**

(30) Priority: 20.12.2022 KR 20220179791
(71) Applicant: Kyung Sang Nam Do, Changwon-si, Gyeongsangnam-do 51154 (KR)
(72) Inventor: AHN, Gwang Hwan, Gimhae-si (KR); CHOI, Seong-Jin, Daegu (KR); KIM, Eun Gyeong, Gimhae-si (KR); KIM, Yeong Bin, Gimhae-si (KR); KIM, Tae Yeup, Gimhae-si (KR); JUNG, Jae Eun, Gimhae-si (KR); LEE, Seon Mi, Gimhae-si (KR); KIM, Young Kwang, Jinju-si (KR); JUNG, Chan Sik, Busan (KR)
(74) Representative: Maiwald GmbH

(57) **Abstract**

Provided are a method of preparing, and use of 1-(2,2-dimethylpropyl)-cyclopropene as an ethylene antagonist that can be formulated as an aqueous emulsion and applicable to plants through immersion or spraying. It was confirmed that the compound can be reliably synthesized and stored by the preparation method disclosed herein, and it was also confirmed that the compound can effectively inhibit ethylene action when formulated as an aqueous emulsion and treated to a plant by immersion or spray treatment; hence, the compound may be used as an ethylene antagonist on plants, as a pre-harvest or post-harvest plant growth regulator, or as a freshness keeping agent.

## Description

### CROSS-REFERENCE TO RELATED APPLICATION

This application is based on and claims priority under 35 U.S.C. §119 to Korean Patent Application No. 10-2022-0179791, filed on December 20, 2022, in the Korean Intellectual Property Office, the disclosure of which is incorporated by reference herein in its entirety.

### BACKGROUND

### 1. Field

The present disclosure relates to a method of preparing, and a use of 1-(2,2-dimethylpropyl)-cyclopropene (1-DCP) as an ethylene antagonist to protect a plant from ethylene action, and which can be formulated as an aqueous emulsion and treated to a plant by immersion or spraying.

### 2. Description of the Related Art

Plants have the ability to produce ethylene on their own, and exogenous or endogenous ethylene has a significant impact on plant growth and development. For example, ethylene in plants may induce physiological activities, such as breaking dormancy of seeds, buds, tuberous roots, tubers, bulbs, and the like; promotion of germination; promotion of the growth of roots and the formation of adventitious roots; inhibition of geotropism; thickening of stems and inhibition of stem elongation; regulation of flower blooming and development of female/male flowers; promotion of dropping of leaves, flowers, and fruits; increase of respiration; increase of autocatalytic production of ethylene; regulation of stress tolerance; regulation of disease resistance or disease tolerance; promotion of pigment formation or breakdown; promotion of fruit softening; and regulation of scent production. Among these actions, ethylene is especially closely related to the senescence of leaves, flowers, stems, etc. or the maturation of fruits, and as such, is also referred to as a plant aging hormone or maturation hormone. Promoting the germination of tuberous roots, tubers, bulbs, promoting dropping of leaves, flowers, and fruits, and promoting fruit softening are representative adverse effects of ethylene that reduce the economic value of plants. Therefore, much research is being conducted on methods of controlling a plant's responses to ethylene for the purpose of inhibiting or blocking ethylene action.

There may be a variety of methods to control the response to ethylene in plants, and one particularly effective method may be to block the action of ethylene using cyclopropene compounds, including 1-MCP. Cycloalkenes in general are in a structurally strained state due to the bond angle between carbons being narrower than that of open-chain hydrocarbons, and in particular, cyclopropene, which has a triangular structure, is known to be the most strained compound among the cycloalkenes. Further, a plant's response to ethylene as a hormone is based on the premise that ethylene binds to the binding sites of ethylene receptor proteins distributed on the cell membrane, and cyclopropene compounds, like ethylene, can also bind to the binding sites of ethylene receptors. However, unlike ethylene, which reversibly binds to the copper ion at the binding site, a cyclopropene compound with a strained structure is known to open its ring structure when binding to the receptor protein, and covalently bonds to amino acid residues at the binding site. This means that, unlike ethylene, a cyclopropene compound irreversibly binds to the binding sites of ethylene receptors and is not re-dissociated. Therefore, the ethylene receptor bound to the cyclopropene compound can no longer bind ethylene, and is therefore excluded from signaling pathways related to ethylene response.

As a result, cyclopropene compounds that bind to ethylene receptors in plants and inhibit the ethylene response can be useful in protecting plants from the undesirable effects of ethylene. Among cyclopropene compounds, 1-MCP (1-methylcyclopropene) in particular, due to having high binding affinity for ethylene receptors, can effectively inhibit ethylene action even at low concentrations, and therefore is widely used as an ethylene antagonist. 1-MCP has a boiling point of 10 °C to 12 °C and exists as a gas at room temperature; and is therefore usually treated as a gas to a target plant. For example, useful is a method that seals a target plant after the harvest in an appropriate space and then exposes the target plant to a specific concentration of 1-MCP gas for a certain period of time. However, to be able to inhibit falling of flowers, leaves, fruits, and the like, delay ripening, suppress senescence, inhibit maturation, and improve storability and the like, also in plants before the harvest in an open space, it requires the use of a compound that remains liquid at room temperature to be applicable by immersion or spraying, and effectively inhibits ethylene action.

In this context, a compound was discovered which can be treated to plants even in an open space and effectively inhibits the ethylene response, and a method of preparing the compound as well as a method of utilizing the compound were developed, thereby completing the present disclosure.

### SUMMARY

One aspect provides a method of producing 1-DCP, the method including: 1) producing α-DIBCl [2-(chloromethyl)-4,4-dimethyl-1-pentene] by reacting α-DIB (α-diisobutylene) with hypochlorite and an acid; 2) producing a 1-DCP [1-(2,2-dimethylpropyl)-cyclopropene] alkali metal salt by reacting the α-DIBCl with an alkali metal alkylamide; and 3) producing 1-DCP by reacting the 1-DCP alkali metal salt with water or an alcohol, wherein the acid includes one or more selected from among hydrochloric acid and a Lewis acid containing chlorine.

The method may include separating and thus obtaining the α-DIB from an α/β-isomer mixture containing α-DIB and β-DIB, wherein the method of separating and obtaining the α-DIB may include the processes of: a) producing β-DIBCl by treating an α/β-isomer mixture containing α-DIB and β-DIB with hypochlorite and an acid; and b) separating α-DIB from a mixture containing the produced β-DIBCI and α-DIB.

Another embodiment provides a 1-DCP [1-(2,2-dimethylpropyl)-cyclopropene] compound represented by Formula 1.

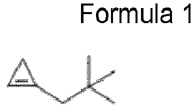

Another aspect provides a 1-DCP inclusion complex, including 1-DCP [1-(2,2-dimethylpropyl)-cyclopropene] and cyclodextrin.

Another aspect provides a method of preparing a 1-DCP inclusion complex, the method including a process of mixing 1-DCP [1-(2,2-dimethylpropyl)-cyclopropene] and cyclodextrin.

Another aspect provides a method of preparing a 1-DCP aqueous-phase emulsion, the method including a process of dissolving a 1-DCP inclusion complex in dimethyl sulfoxide (DMSO).

Another aspect provides an ethylene response-inhibiting composition including 1-DCP [1-(2,2-dimethylpropyl)-cyclopropene], a hydrate thereof, a solvate thereof, a salt thereof, or a complex thereof.

Another aspect provides a method of inhibiting ethylene response in a plant, the method including a process of applying a composition to a plant, the composition including 1-DCP [1-(2,2-dimethylpropyl)-cyclopropene], a hydrate thereof, a solvate thereof, a salt thereof, or a complex thereof.

Additional aspects will be set forth in part in the description which follows and, in part, will be apparent from the description, or may be learned by practice of the presented embodiments of the disclosure.

### A. Preparation method of 1-DCP

One aspect provides a method of producing 1-DCP, the method including: 1) producing α-DIBCl [2-(chloromethyl)-4,4-dimethyl-1-pentene] by reacting α-DIB (α-diisobutylene) with hypochlorite and an acid; 2) producing an alkali metal salt of 1-DCP [1-(2,2-dimethylpropyl)-cyclopropene] by reacting the α-DIBCl with an alkali metal alkylamide; and 3) producing 1-DCP by reacting the 1-DCP alkali metal salt with water or an alcohol, wherein the acid includes one or more selected from among hydrochloric acid and a Lewis acid containing chlorine.

As used herein, the term "1-DCP [1-(2,2-dimethylpropyl)-cyclopropene]" is a cyclopropene compound, which is represented by Formula 1 below.

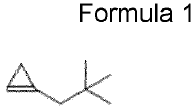

In the above method, the process 1), as a process of reacting α-DIB (α-diisobutylene) with hypochlorite and an acid to produce α-DIBCl [2-(chloromethyl)-4,4-dimethyl-1-pentene], may include a process such as Reaction Scheme A below.

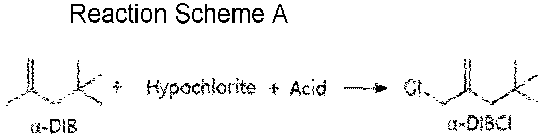

Specifically, the process 1), as a method of producing and obtaining α-DIBCl, may include the processes of: a) mixing α-DIB with hypochlorite and an acid to chlorinate α-DIB to α-DIBCl (allylic chlorination); and b) separating/obtaining α-DIBCl from the reaction product.

The α-DIB (α-diisobutylene) may be represented by Formula 2 below.

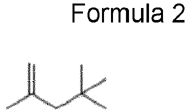

The acid may include one or more selected from hydrochloric acid or a Lewis acid containing chlorine. Further, the Lewis acid may include one or more selected from FeCl₃, AlCl₃, CeCl₃, and MoCl₅.

According to an embodiment, the acid may include one or more selected from hydrochloric acid, FeCl₃, AlCl₃, CeCl₃, and MoCl₅, and for example, may be hydrochloric acid.

The hypochlorite may be an alkali metal hypochlorite or an alkaline earth metal hypochlorite, and for example, may be one selected from sodium hypochlorite (NaOCI), potassium hypochlorite (KOCI), lithium hypochlorite (LiOCI), and calcium hypochlorite [Ca(OCl)₂].

According to an embodiment, the hypochlorite may be one or more selected from sodium hypochlorite (NaOCI), potassium hypochlorite (KOCI), lithium hypochlorite (LiOCI), and calcium hypochlorite [Ca(OCl)₂], and for example, may be sodium hypochlorite or calcium hypochlorite.

In the process 1), the mixing ratio (mol) of α-DIB and hypochlorite may be about 1:0.2 to 2, and may be, for example, about 1:0.2, about 1:0.4, about 1:0.6, about 1:0.8, about 1:1, about 1:1.2, about 1:1.4, about 1:1.6, about 1:1.8, or about 1:2.

In the process 1), the mixing ratio (mol) of hypochlorite and acid may be about 1.2:0.5 to 2, and may be, for example, about 1.2:0.5, about 1.2:0.7, about 1.2:0.9, about 1.2:1.1, about 1.2:1.3, about 1.2:1.5, about 1.2:1.7, about 1.2:1.9, or about 1.2:2.

In an embodiment, the mixing ratio (in moles) of α-DIB, hypochlorite and acid may be about 1:1.2:1.3.

In the process b), the method of separating/obtaining α-DIBCl from the reaction product may be one of chromatography, a solvent extraction method, and a fractional distillation method, and may be, for example, a fractional distillation method.

The α-DIB may be a purchased pre-made product or may be obtained from an α/β-isomer mixture containing α-DIB and β-DIB by separation, and the method may include the processes of: a) producing β-DIBCI by treating an α/β-isomer mixture containing α-DIB and β-DIB with hypochlorite and an acid; and b) separating α-DIB from a mixture containing the produced β-DIBCI and α-DIB.

In particular, the method of separating and obtaining the α-DIB may include the processes of a) selectively converting β-DIB to β-DIBCI (1-chloro-2,4,4-trimethyl-2-pentene) by mixing an α/β-isomer mixture containing α-DIB and β-DIB with hypochlorite and an acid; and b) separating/obtaining α-DIB from a mixture of α-DIB and β-DIBCl, and may be carried out through a process such as Reaction Scheme B below.

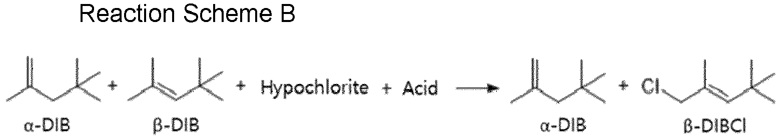

The acid may include one or more selected from hydrochloric acid or a Lewis acid containing chlorine. Further, the Lewis acid may include one or more selected from FeCl₃, AlCl₃, CeCl₃, and MoCl₅.

According to an embodiment, the acid may include one or more selected from hydrochloric acid, FeCl₃, AlCl₃, CeCl₃, and MoCl₅, and for example, may be hydrochloric acid.

The hypochlorite may be an alkali metal hypochlorite or an alkaline earth metal hypochlorite, and for example, may be one selected from sodium hypochlorite (NaOCI), potassium hypochlorite (KOCI), lithium hypochlorite (LiOCI), and calcium hypochlorite [Ca(OCl)₂].

According to an embodiment, the hypochlorite may be one or more selected from sodium hypochlorite (NaOCI), potassium hypochlorite (KOCI), lithium hypochlorite (LiOCI), and calcium hypochlorite [Ca(OCl)₂], and for example, may be sodium hypochlorite or calcium hypochlorite.

In the above process, the mixing ratio (mol) of DIB (α/β-isomer mixture) and hypochlorite may be about 2:0.5 to 2, and for example, may be about 2:0.5, about 2:0.7, about 2:0.9, about 2:1.1, about 2:1.3, about 2:1.5, about 2:1.7, about 2:1.9, or about 2:2.

In the above process, the mixing ratio (mol) of hypochlorite and acid may be about 1:0.5 to 2, and for example, may be about 1:0.5, about 1:0.7, about 1:0.9, about 1:1, about 1:1.2, about 1:1.4, about 1:1.6, about 1:1.8, or about 1:2.

In an embodiment, the mixing ratio (mol) of DIB, hypochlorite, and acid may be about 2:1:1.

In the process b), the method of separating/obtaining α-DIB from a mixture of α-DIB and β-DIBCI may be one of chromatography, a solvent extraction method, and a fractional distillation method, and may be, for example, a fractional distillation method.

In the method, the process 2), as a process of reacting the α-DIBCl prepared above with an alkali metal alkylamide to produce a 1-DCP [1-(2,2-dimethylpropyl)-cyclopropene] alkali metal salt, may be performed through a process such as Reaction Scheme C below. Further, the process 2) may further include a process of separating and thus obtaining the produced 1-DCP alkali metal salt.

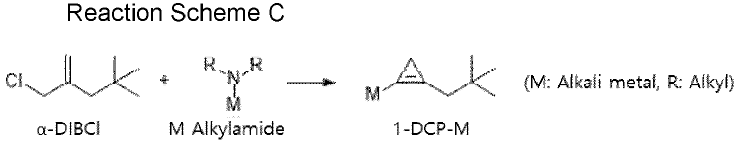

Specifically, the process 2) may include the following processes: a) producing a 1-DCP alkali metal salt by cyclopropenation of α-DIBCl using an alkali metal alkylamide; and b) separating the 1-DCP alkali metal salt from a reaction product.

The α-DIBCl may be expressed by Formula 3 below.

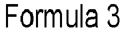

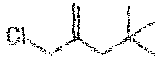

The alkali metal may be lithium (Li), sodium (Na), or potassium (K), and may be, for example, lithium.

Thus, the 1-DCP alkali metal salt produced in the above reaction may be one or more selected from a 1-DCP lithium salt, a 1-DCP sodium salt, and a 1-DCP potassium salt, and may be, for example, a 1-DCP lithium salt. The 1-DCP lithium salt {[2-(2,2-dimethylpropyl)cycloprop-1-en-1-yl]lithium} may be expressed by Formula 4 below.

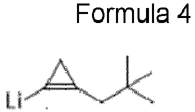

The process 2) may include a process of obtaining the prepared 1-DCP alkali metal salt by separation, and in particular, the prepared 1-DCP alkali metal salt may be obtained by being separated from reactants and by-products of the process 2) through a separate process, and the process 3) may be performed by using the separated 1-DCP alkali metal salt.

According to an embodiment, if 1-DCP is to be prepared by directly reacting a 1-DCP alkali metal salt prepared by reacting α-DIBCl with an alkali metal alkylamide, α-DIB produced during the synthesis process may remain, and since the α-DIB is not removable by methods such as distillation, the purity of the final 1-DCP product may decrease. Therefore, if the 1-DCP alkali metal salt prepared as above is obtained by separation and 1-DCP is prepared based on the separately separated 1-DCP alkali metal salt, the product with significantly superior purity may be obtained.

The alkali metal alkylamide may include one or more selected from lithium diethylamide, lithium diisopropylamide, sodium diethylamide, sodium diisopropylamide, potassium diethylamide, and potassium diisopropylamide, and may be, for example, lithium diethylamide (LDEA) or lithium diisopropylamide (LDA).

The alkali metal alkylamide may be a pre-made product or may be synthesized.

The process of synthesizing the alkali metal alkylamide may be performed as shown in Reaction Scheme D below.

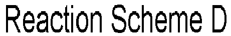

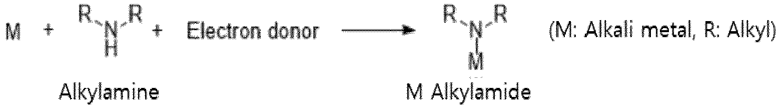

The above process may be a process of synthesizing an alkali metal alkylamide (in particular, lithium alkylamide) by reacting an alkali metal (in particular, lithium) and an alkylamine with an electron donor. The alkylamine may be one of diethylamine (DEA) and diisopropylamine, and may be, for example, DEA. Additionally, the electron donor may be one of isoprene (ISP) and styrene, and may be, for example, ISP.

In the process b), the method of separating a 1-DCP alkali metal salt (in particular, a lithium salt) from the reaction product may include a method that includes filtration of the reaction product and evaporating the filtrate under reduced pressure by using a rotary evaporator.

In the above method, the process 3) as a process of preparing 1-DCP from the 1-DCP alkali metal salt, may include a process as shown in Reaction Scheme E below.

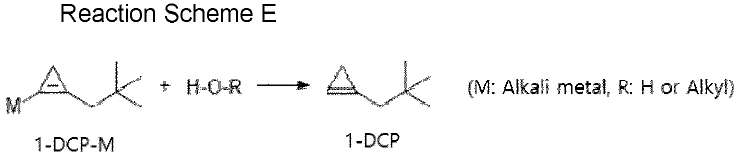

The method of producing 1-DCP may include the following processes: a) producing 1-DCP by reacting, i.e. neutralizing a 1-DCP alkali metal salt (in particular, 1-DCP lithium salt) with water or an alcohol; and b) separating and recovering the produced 1-DCP, followed by purification by distillation.

The process may include a process of washing a reaction product of neutralization of the 1-DCP alkali metal salt, and in particular, may involve washing with hydrochloric acid and/or salt water.

The process may include a process of separating/recovering 1-DCP from a reaction product of neutralization of the 1-DCP alkali metal salt, and in particular, may involve separating 1-DCP through vacuum distillation.

### B. 1-DCP [1-(2,2-dimethylpropyl)-cyclopropene] compound

Another embodiment may provide a 1-DCP [1-(2,2-dimethylpropyl)-cyclopropene] compound represented by Formula 1. The same parts as described above also apply to the above compound:

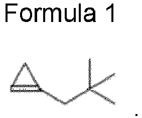

The 1-DCP compound may be prepared using the preparation method described above.

### C. Preparation Method of 1-DCP Inclusion Complex

Another aspect provides a method of preparing a 1-DCP inclusion complex, the method including mixing 1-DCP [1-(2,2-dimethylpropyl)-cyclopropene] and cyclodextrin. The same parts as described above also apply to the above method.

The method, as a process of preparing a stable inclusion complex from 1-DCP, may include a process as shown in Reaction Scheme F below. It should be noted that the cyclodextrin expressed in the following reaction scheme is shown as a schematic diagram, not a chemical formula.

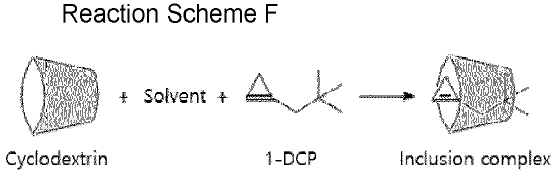

The method of preparing the inclusion complex may include the following processes: a) after dissolving cyclodextrin in a solvent, including 1-DCP in the cyclodextrin; and b) separating an inclusion complex from the solvent, followed by drying and forming as solid powder.

In the process a), the cyclodextrin may be one of α-cyclodextrin, β-cyclodextrin, or gamma-cyclodextrin, or one of derivatives thereof, and may be, for example, α-cyclodextrin. Additionally, the cyclodextrin may include a modified cyclodextrin as well as a cyclodextrin mixture and a cyclodextrin polymer. The solvent for dissolving cyclodextrin may be one of water, urea solution, and dimethyl sulfoxide (DMSO), and in particular, may be water.

In the process b) above, the method for separating the inclusion complex may be one of centrifugation and vacuum filtration, and in particular, may be a vacuum filtration method. Further, the drying method may be one of room-temperature drying, hot-air drying, and vacuum drying, and in particular, may be a vacuum drying method.

### D. 1-DCP inclusion complex

Another aspect provides a 1-DCP inclusion complex, including 1-DCP [1-(2,2-dimethylpropyl)-cyclopropene] and cyclodextrin. The same parts as described above also apply to the above complex.

As used herein, the term "inclusion complex" refers to a complex compound formed by accommodating a guest compound with suitable dimensions and shape in the 1-3 dimensional molecular-scale space formed by a host compound, and is also known as host-guest compound and inclusion compound.

The 1-DCP inclusion complex refers to a combination of 1-DCP (guest compound) and cyclodextrin (host compound), wherein 1-DCP is positioned substantially inside the internal cavity of the cyclodextrin ring. A complexed 1-DCP compound must meet a size criteria to fit at least partially into the internal cavity of cyclodextrin to form an inclusion complex.

The cyclodextrin may be one of α-cyclodextrin, β-cyclodextrin, or gamma-cyclodextrin, or one of derivatives thereof, and may be, for example, α-cyclodextrin. Additionally, the cyclodextrin may include a modified cyclodextrin as well as a cyclodextrin mixture and a cyclodextrin polymer.

The 1-DCP inclusion complex may be expressed by Formula 5. It should be noted that the cyclodextrin expressed in the following Formula is shown as a schematic diagram, not a chemical formula.

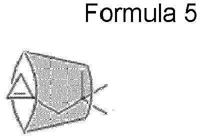

The 1-DCP inclusion complex may be prepared by the preparation method of a 1-DCP inclusion complex described above.

### E. Preparation method of 1-DCP aqueous emulsion

Another aspect is to provide a method of preparing a 1-DCP aqueous emulsion, the method including a process of dissolving a 1-DCP inclusion complex in dimethyl sulfoxide (DMSO). The same parts as described above also apply to the above method.

The method may include, as a process of dissolving a poorly-soluble 1-DCP inclusion complex to prepare a 1-DCP aqueous emulsion, a process as shown in Reaction Scheme G below. It should be noted that the cyclodextrin expressed in the following reaction scheme is shown as a schematic diagram, not a chemical formula.

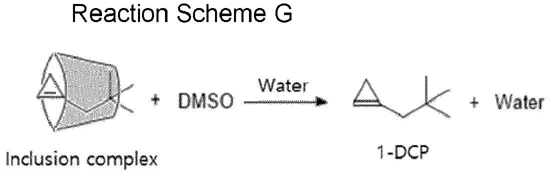

The method of preparing the 1-DCP aqueous emulsion may include the following processes: a) dissolving the inclusion complex in dimethyl sulfoxide (DMSO) to prepare a 1-DCP/DMSO solution; and b) mixing the prepared 1-DCP/DMSO solution with water to form 1-DCP aqueous emulsion having an appropriate concentration.

In the process a), the ratio (weight/volume) of inclusion complex to DMSO may be about 1:1 to 4, for example, about 1:1, about 1:1.2, about 1:1.4, about 1:1.6, about 1:1.7, about 1:1.8, about 1:2, about 1:2.2, about 1:2.4, about 1:2.5, about 1:2.6, about 1:2.8, about 1:3, about 1:3.2, about 1:3.4, about 1:3.6, about 1:3.8, about 1:4, and more specifically, may be about 1:2.5.

In the process b), the mixing ratio of water to 1-DCP/DMSO may be about 100 times to about 1,000 times (by volume).

For example, the 1-DCP aqueous emulsion may contain a surfactant.

### F. Composition for inhibiting ethylene response in plants

Another aspect may provide a composition for inhibiting ethylene response in a plant, the composition including 1-DCP [1-(2,2-dimethylpropyl)-cyclopropene], a hydrate thereof, a solvate thereof, a salt thereof, or a complex thereof. The same parts as described above also apply to the above composition.

The composition may serve to inhibit ethylene response in a plant as 1-DCP [1-(2,2-dimethylpropyl)-cyclopropene], a hydrate thereof, a solvate thereof, a salt thereof, or a complex thereof included in the composition acts on the plant as an active ingredient. For example, the composition may be a growth regulator or a freshness retainer.

The complex may include a 1-DCP inclusion complex, wherein the inclusion complex may be dissolved in a solvent to release an active compound from the inclusion complex, after which the compound acts upon contact with the plant.

As used herein, the term "ethylene" refers to an unsaturated hydrocarbon in a gaseous state with a C₂H₄ structure. Exogenous ethylene, or endogenous ethylene produced by plants on their own has a significant impact on the growth and development of the plants, and is closely related to the senescence of leaves, flowers, stems, etc. and the maturation of fruits, and as such, is also referred to as a plant aging hormone or maturation hormone.

The ethylene may induce physiological activities in plants, such as promotion of germination and breaking dormancy of seeds, buds, tuberous roots, tubers, bulbs, and the like; promotion of root growth and adventitious root formation; inhibition of geotropism; thickening of stems and inhibition of stem elongation; regulation of flower blooming and development of female/male flowers; promotion of dropping of leaves, flowers, and fruits; increase of respiration; increase of autocatalytic production of ethylene; regulation of stress tolerance; regulation of disease resistance or disease tolerance; promotion of pigment formation or breakdown; promotion of fruit softening; and regulation of scent production. For example, the ethylene response inhibition may be inhibition of one or more selected from the aforementioned physiological activities.

The composition may be for regulating one or more physiological activities in a plant, from among germination and breaking dormancy of seeds, buds, tuberous roots, tubers, bulbs, and the like, root growth and adventitious root formation, geotropism, stem elongation and thickening, flower blooming and development of female/male flowers, dropping of leaves, flowers, and fruits, respiration, ethylene production, stress tolerance, disease resistance or disease tolerance, formation or breakdown of pigments, fruit softening, and scent production.

The composition may be a solid preparation, a liquid preparation, or a gaseous preparation. The solid, liquid and gaseous preparations may be prepared by various methods in the related art. For example, the preparations may be formulated by blending active ingredients with a solid carrier in the form of powder, with a liquid carrier in the form of mixtures, solutions, dispersions, emulsions, and suspensions, or with a volatile liquid or gaseous carrier in the form of an aerosol.

### G. Methods for inhibiting ethylene response in plants

Another aspect is to provide a method of inhibiting ethylene response in a plant, the method including a process of applying a composition to a plant, the composition including 1-DCP [1-(2,2-dimethylpropyl)-cyclopropene], a hydrate thereof, a solvate thereof, a salt thereof, or a complex thereof. The same parts as described above also apply to the above method.

As used herein, the term "plant" is used in its general sense of the word and includes, for example, herbaceous and woody plants. A plant treated by the method disclosed herein may include a whole or part of the plant body, such as seeds, bulbs, tubers, roots, stems, leaves, flowers, fruits, and parts thereof.

For example, the plant may be one of food crops, medicinal crops, specialty crops, edible horticultural crops, ornamental horticultural crops, and landscaping crops.

The composition may be applied to a plant by a variety of suitable means. For example, application of the composition, alone or in combination with a carrier, may be conducted by contacting the plant to be treated with the composition. In particular, the method may involve bringing the composition in the form of a single or combined preparation into contact with a plant as a gaseous form by vaporization, bringing the composition in the form of a single or combined preparation into contact with a plant as a solid powder preparation through spraying or coating, or bringing the composition in the form of a single or combined preparation into contact with a plant as a liquid preparation by means of immersion or spraying in an open or a sealed spaces.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 shows the results of mass spectrum analysis of α-DIB;
FIG. 2 is a chromatogram of gas chromatography analysis of intermediates and products during the process of separating/obtaining α-DIB from an α/β-isomer mixture of DIB;
FIG. 3 shows the results of mass spectrum analysis of α-DIBCl;
FIG. 4 is a chromatogram of gas chromatography analysis of intermediates and products during the process of producing α-DIBCl from α-DIB;
FIG. 5 shows the results of mass spectrum analysis of 1-DCP;
FIG. 6 is a chromatogram of gas chromatography analysis of intermediates and products during the process of preparing 1-DCP from a 1-DCP lithium salt;
FIG. 7 is a diagram showing the process from LDEA synthesis to 1-DCP synthesis in Example 1;
FIG. 8 is a diagram showing the process of preparing an inclusion complex by incorporating 1-DCP into α-cyclodextrin in Example 2;
FIG. 9 is a diagram showing the effect of inhibiting the maturation of banana fruit, following an immersion treatment of 1-DCP aqueous emulsion;
FIG. 10 is a diagram showing the effect of suppressing the softening of Taechu sweet persimmon fruits, following a spray treatment of 1-DCP aqueous emulsion; and
FIG. 11 is a diagram showing the results of measuring a change in flesh hardness of Taechu sweet persimmon fruits, following a spray treatment with 1-DCP aqueous emulsion.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Further descriptions will be provided in greater detail through examples below. However, these examples are for illustrative purposes only, and the scope of the present disclosure is not limited to these examples.

Unless stated otherwise, the materials used in the following Examples were purchased from commercial suppliers and used without further purification. All solvents were treated by a common method. Diisobutylene (DaeJung Chemicals and Metals, Korea), calcium hyperchlorite (Ca(OCl)₂, Samchun Chemicals, Korea), hydrochloric acid (Daejung), lithium (1-6 mm particles, Thermo-Fisher Scientific, USA), diethylamine (Daejung), isoprene (Samchun), diethyl ether (Daejung), Celite 545, and cyclodextrin (Henrikang Biotech, China) were obtained as commercially available reagent-grade products. Nuclear magnetic resonance (¹H NMR and ¹³C NMR) spectra were obtained by Bruker-500 MHz spectroscopy in a CDCl₃ solution. The NMR chemical shift of residual CDCl₃ (7.25 ppm) was recorded. Proton-decoupled ¹³C NMR spectra were obtained with respect to a chemical shift of CDCl₃ (77.0 ppm). Chromatograms were obtained using a gas chromatograph (Shimadzu GC 2010, Japan) equipped with a flame ionization detector (FID) and a capillary column (HP-5, non-polar, 0.25 mm x 30 m, FT = 0.25 µm). The column temperature was maintained at 60 °C for 2 minutes, and then increased to 200 °C at 25 °C/min. Mass spectra were obtained using a gas chromatograph (Thermo-Fisher, TRACE 1610) connected to a mass spectrometer (ISQ7610) and equipped with a TraceGold-5SiMS capillary column (non-polar, 0.25 mm x 30 m, film thickness = 0.25 mm). The oven temperature program started at 50 °C, then gradually increased to 300 °C at 10 °C/min and maintained at 300 °C for 10 minutes.

### Example 1: Synthesis of 1-DCP

The present disclosure relates to a method of synthesizing 1-(2,2-dimethylpropyl)-cyclopropene [1-DCP], 1-DCP synthesized by the method, and uses thereof, wherein the entire process of 1-DCP synthesis may be summarized as Reaction Scheme 1 below, the process of which is described in greater detail below.

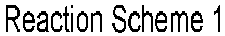

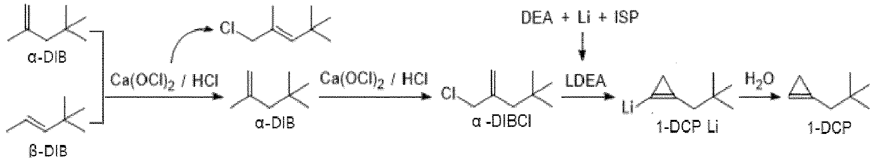

### 1-1: Separation/Obtaining of α-DIB from α/β-DIB mixture

The process of separating/obtaining α-DIB from the α/β-DIB mixture may be omitted by purchasing and using a pre-made α-DIB product. Diisobutylene (DIB) is commercially available as a 3:1 mixture of α/β-isomers (CAS 25167-70-8) or as an α-DIB isolate (CAS 107-39-1), wherein the isolate is 10 times more expensive than the mixture. Therefore, separating/obtaining the isolate from the mixture may be more economical than purchasing the isolate. Further, the use of mixture DIB (CAS 107-39-1) in the synthesis of 1-DCP significantly decreases the purity and yield of the final product 1-DCP, and is thus not practical.

Because the difference in boiling point between α- and β-isomers of DIB is only 1°C to 2°C, the two isomers cannot be separated through distillation. However, it was discovered that when the α/β-DIB mixture is chlorinated using an appropriate ratio of hypochlorite, β-DIB is chlorinated before α-DIB, and converted to β-DIBCl, which has a much higher boiling point. Therefore, exhausting β-DIB by converting the same to β-DIBCl may allow recovery of α-DIB from the mixture of α-DIB and β-DIBCI through the difference in boiling point.

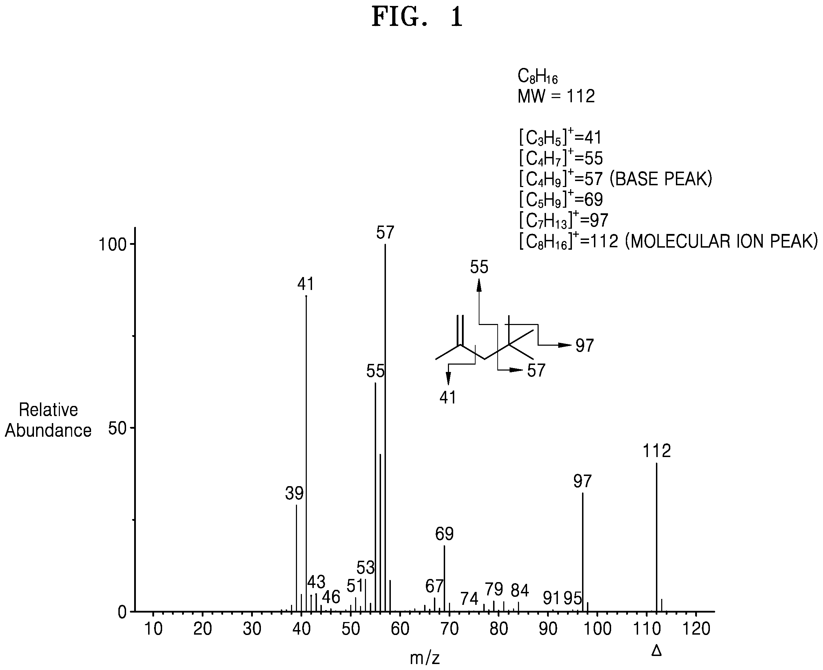

The method by which α-DIB was separated/obtained from the α/β-DIB mixture may be summarized as Reaction Scheme 2 below, the process of which is described in greater detail below. 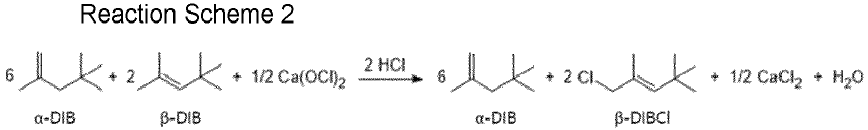
(1) Preparation of hypochlorite aqueous solution: 200 g of calcium hypochlorite (Ca(OCl)₂, 70 % purity, Samchun Chemicals) were added to 1 L of distilled water and subjected to ultrasonification for 30 minutes, then through centrifugation or refrigeration overnight to settle insoluble precipitates, the supernatant was collected to prepare a hypochlorite aqueous solution. The aqueous hypochlorite solution was refrigerated and cooled, before use.
(2) In a 1 L pressure-resistant bottle (maxP=1.5 bar) (Duran, Pressure plus bottle, code 10130780), 328 mL of cooled DIB (2 mol) (diisobutylene, 3:1 mixture of α-/β-isomer, Daejeong Chemicals) and 511 mL of a cooled aqueous hypochlorite solution [1 mol (OCl)⁻] were added.
(3) In the pressure-resistant bottle, 85.8 mL of cooled concentrated hydrochloric acid (1 mol) (Daejeong Chemicals) was added, and the bottle was tightly closed with a stopper and vigorously shaken to mix the reactants. During the mixing process, chlorination of β-DIB takes place; this reaction is completed within 1 minute, and whether the reaction is complete is confirmed by a change in the color of the reaction solution (green to colorless). Here, the hydrochloric acid must be placed in a separate isolation container before addition; this is to ensure that the hydrochloric acid added in the pressure-resistant bottle comes into contact with hypochlorite only after the stopper is closed. If hypochlorite comes into contact with the hydrochloric acid before closing the stopper, a large amount of chlorine gas may be emitted with no chlorination reaction taking place. Caution must be exercised as high temperatures (60 °C to 70 °C) and high pressures (1.5 atm or less) are formed during the reaction process.
(4) The reactants were transferred to a 1 L separatory funnel to remove the bottom layer (aqueous phase), and the collected supernatant was refrigerated for 1 hour to further remove a small amount of settled water to obtain crude α-DIB (328 mL, colorless). The above process was repeated 6 times to obtain about 2 L of crude α-DIB.
(5) First fractional distillation: A fractional distillation system was constructed by equipping a 1 L two-necked round flask with a stirring magnet, a Liebig condenser (cooling water temperature 0 °C), a 30 cm Vigreux column, a 200 °C thermometer, a heating mantle, and a receiver (500 mL mass cylinder). 1 L of crude α-DIB obtained in the above process was added to the flask, followed by heating and distillation. Here, the solution temperature was maintained at 116 °C to 140 °C, and the steam temperature increased to 100 °C to 105 °C, and then decreased down to 90 °C to 80 °C. In the above process, the first fractional distillation was terminated if 50 % of the administered amount was recovered as distillate (500 mL recovered, colorless, purity 93 %). The above process was repeated 2 times to obtain 1 L of first distilled α-DIB.
(6) Second fractional distillation: to perform a second fractional distillation of the first distilled α-DIB obtained in the above process, 1L of first distilled α-DIB was placed into a fractional distillation system, and distilled by heating. Here, the solution temperature was maintained at 103 °C to 130 °C, and the steam temperature increased to 40 °C to 93 °C, and then decreased. The heating temperature was adjusted to maintain a distillation rate of 7-8 mL/min. In the above process, the second fractional distillation was terminated if 90 % of the administered amount was recovered as distillate (900 mL α-DIB, colorless, d=0.73, purity 98 %, final yield 63 %). ¹ H NMR (500 MHz, CDCl₃) δ 4.83 (dd, J = 2.5, 1.4 Hz, 1H), 4.63 - 4.61 (m, 1H), 1.93 (s, 2H), 1.77 (s, 3H), 0.92 (s, 9H). ¹³C NMR (126 MHz, CDCl₃) δ144.02, 113.73, 51.66, 31.35, 30.06, 25.27. GCMS [C₃H₅]⁺=41, [C₄H₇]⁺=55, [C₄H₉]⁺=57 base peaks, [C₅H₉]⁺=69, [C₇H₁₃]⁺=97, [C₈H₁₆]⁺=112 molecular ion peaks Mass spectrum analysis of α-DIB showed a molecular ion peak ([C₈H₁₆]⁺=112) and a base peak ([C₄H₉]⁺=57) (FIG. 1).

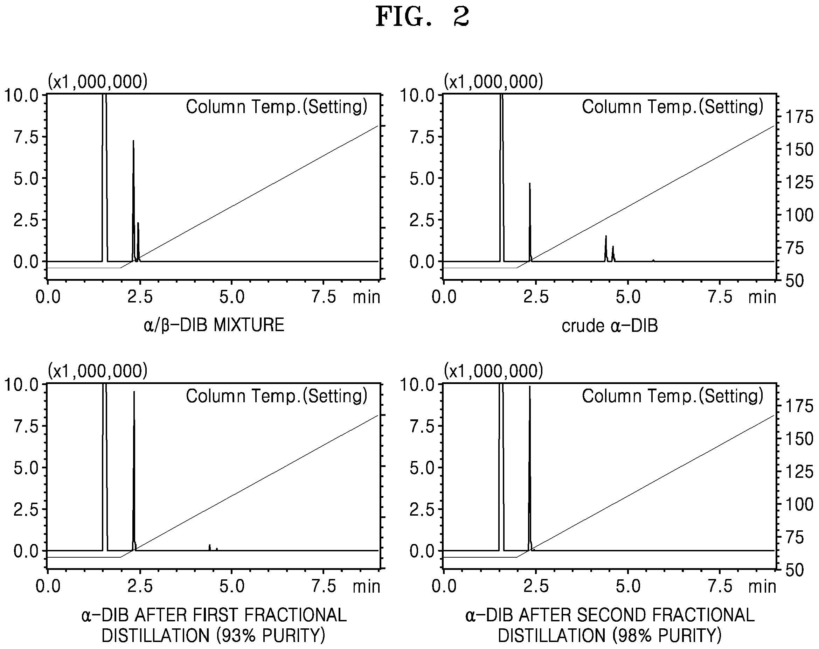

During the above process, reaction intermediates were tested for purity through gas chromatography analysis (FIG. 2).

In the above process, Ca(OCl)₂ can be replaced with other hypochlorite such as NaOCI, but using Ca(OCl)₂ may be economical, and hydrochloric acid can be replaced with a Lewis acid containing chlorine (e.g., FeCl₃ and AlCl₃), but using hydrochloric acid may be economical.

### 1-2: α-DIBCl synthesis

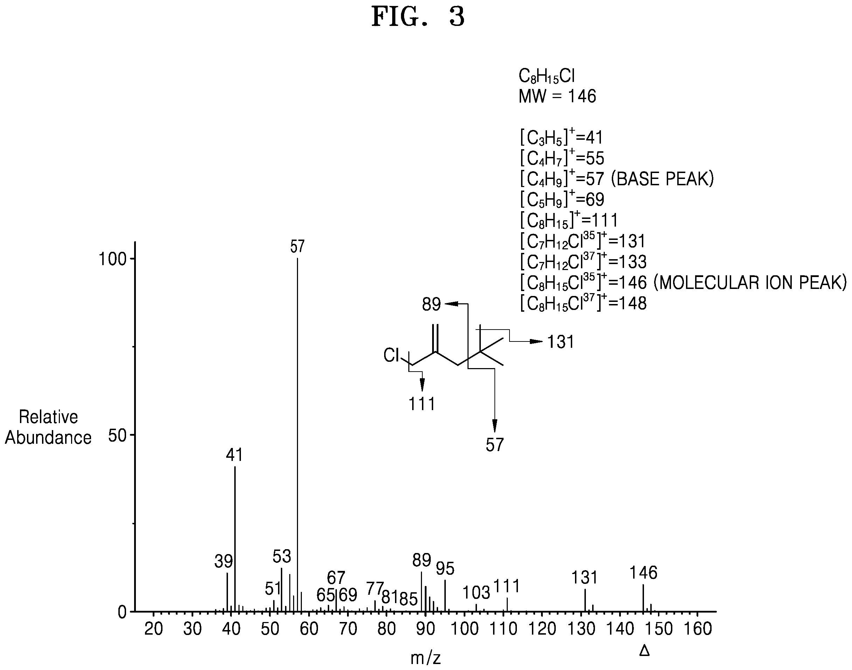

The method by which α-DIBCl was synthesized from α-DIB may be summarized as Reaction Scheme 3 below, the process of which is described in greater detail below. 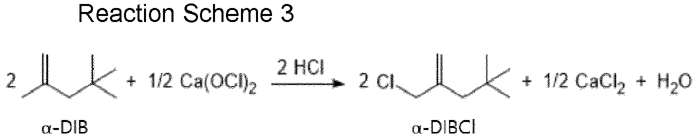
(1) Preparation of an aqueous hypochlorite solution: the same process as the process 1) of 1-1 above.
(2) In a 1 L pressure-resistant bottle (maxP=1.5 bar) (Duran, Pressure plus bottle, code 10130780), 157 mL of cooled α-DIB (1 mol) and 613 mL of a cooled aqueous hypochlorite solution [1.2 mol (OCI)-] were added. Here, the solution must be cool (0-4 °C); if the solution is not cool, α-DIB may remain in the solution later.
(3) In the pressure-resistant bottle, 114 mL of cooled concentrated hydrochloric acid (1.3 mol) (Daejeong Chemicals) was added, and the bottle was tightly closed with a stopper and vigorously shaken to mix the reactants. During the mixing process, allylic chlorination of α-DIB takes place; this reaction is completed within 1 minute, and whether the reaction is complete is confirmed by a change in the color of the reaction solution (green to colorless). Here, the hydrochloric acid must be placed in a separate isolation container before addition; this is to ensure that the hydrochloric acid added in the pressure-resistant bottle comes into contact with hypochlorite only after the stopper is closed. If hypochlorite comes into contact with the hydrochloric acid before closing the stopper, a large amount of chlorine gas may be emitted with no chlorination reaction taking place. Caution must be exercised as high temperatures (60 °C to 70 °C) and high pressures (1.5 atm or less) are formed during the reaction process.
(4) The above reactants were transferred to a 1 L separatory funnel, and the supernatant recovered after removing the bottom layer (aqueous phase) was subject to centrifugation (3,000 rpm to 5,000 rpm, 3 minutes to 5 minutes) to further remove a small amount of water settled, thereby obtaining crude α-DIBCl (160 mL, colorless, yield 67 %). Here, dehydration can be performed instead of centrifugation, by administering anhydrous sodium sulfate powder. The above process was repeated 5 times to obtain 800 mL of crude α-DIBCl.
(5) Fractional distillation: A fractional distillation system was constructed by equipping a 1 L two-necked round flask with a stirring magnet, a Liebig condenser (cooling water temperature 0 °C), a 30 cm Vigreux column, a 200 °C thermometer, a heating mantle, and a receiver (500 mL mass cylinder). 800 mL of crude α-DIBCl obtained in the above process was added to the flask, followed by heating and distillation. Here, the solution temperature was maintained at 100 °C to 120 °C, and the steam temperature increased to 80 °C to 85 °C, and then decreased. In the above process, the distillation was terminated when 70 % of the administered amount was recovered as distillate (560 mL α-DIBCl, colorless, purity 80 %, yield 78 %). α-DIBCl ¹HNMR (500MHz, CDCl₃) δ 5.28 (d, J = 1.1 Hz, 1H), 4.96 (s, 1H), 4.07 (d, J = 0.9 Hz, 2H), 2.09 (s, 2H), 0.93 (s, 9H). ¹³C NMR (126 MHz, CDCl₃) δ 143.24, 118.11, 49.78, 46.35, 31.43, 29.75. Mass spectrum analysis of α-DIBCl showed a molecular ion peak ([C₈H₁₅Cl]⁺=146) and a base peak ([C₄H₉]⁺=57) (FIG. 3).

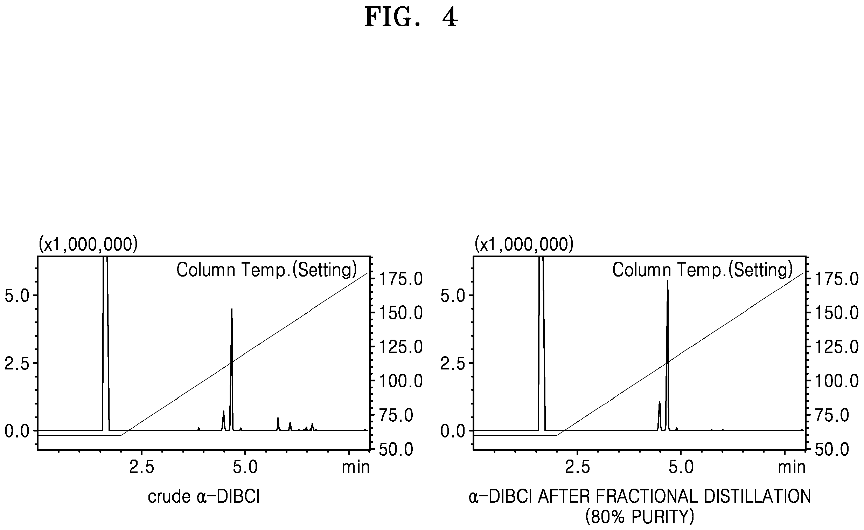

In particular, the purity of the product of the above process was confirmed through gas chromatography analysis (FIG. 4).

In the above process, Ca(OCl)₂ can be replaced with other hypochlorite such as NaOCI, but using Ca(OCl)₂ may be economical, and hydrochloric acid can be replaced with a Lewis acid containing chlorine (e.g., FeCl₃ and AlCl₃), but using hydrochloric acid may be economical.

### 1-3: 1-DCP synthesis

Cyclopropene is generally synthesized through α-elimination which removes alpha halogen from allylic halide by a strong base. The strong base used in this reaction is usually alkali metal salts of amines, including sodium amide and lithium diisopropylamide (LDA). For example, 1-methylcylopropene (1-MCP) may be synthesized from β-methallyl chloride by using the sodium amide or LDA. However, it was found that under the conditions for 1-DCP synthesis, α-DIBCl does not react with sodium amide but is cyclopropenated by a metal salt of an amide containing an alkyl group.

The method for synthesizing 1-DCP from α-DIBCl may be summarized in Reaction Schemes 4 to 6 below. Here, the process of lithium diethylamide (LDEA) synthesis in Reaction Scheme 4 below is a process for synthesizing an alkylamide metal salt, and may be omitted by purchasing and using a pre-made commercial LDA product. However, unlike commercially available LDA, LDEA may not be commercially available, and the use of LDEA may be advantageous over using LDA in terms of improving the purity of the final product, 1-DCP. This is because the reaction by-products of LDEA due to having a lower boiling point than the reaction by-products of LDA and thus may be more easily removed during the purification process of 1-DCP. Since commercial LDA contains a solvent with a high boiling points (e.g., cyclohexane, etc.) or reaction by-products (e.g., ethyl benzene), the use of commercial LDA may make the purification process extremely difficult. Further, synthesizing LDEA may be more economical than obtaining LDA. The specific method for synthesizing 1-DCP from α-DIBCl was as follows.

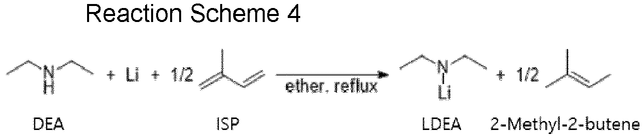

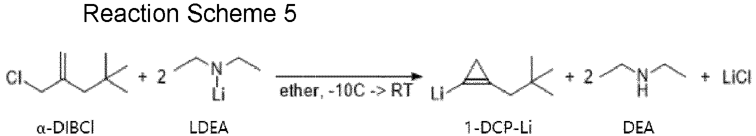

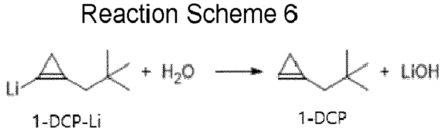

### (1) Synthesis of LDEA (Reaction Scheme 4)

1) A reaction system was constructed by equipping a 500 mL three-necked round flask with a Dimroth condenser (cooling water temperature 0 °C) / gas bubbler, a 100 °C thermometer, and a heating mantle.
2) In a flask, 3.51 g of lithium (0.5 mol) (1-6 mm granules, Alfa Aesar), 52.78 mL of DEA (0.5 mol) (diethylamine, Daejung Chemical), 200 mL of diethyl ether (Daejung Chemical) were added with a stirring magnet. Here, lithium was withdrawn from the opened lithium reagent bottle and weighed quickly while blowing Ar gas, and once the lithium was placed in the flask, the mouth of the flask was quickly closed with a rubber septum, and a syringe was inserted therein to flow Ar gas therein. This is because lithium, if exposed to air for an extended period of time, reacts with oxygen and moisture and becomes discolored, which may retard the synthesis of LDEA and cause impurity.
3) After flowing Ar gas into the flask for 2 minutes to 3 minutes to expel air, while vigorously stirring the reactants at 800 rpm to 1,000 rpm using a stirring magnet, 27.79 ml of ISP (0.275 mol) (isoprene, Samchun Chemicals) was added through the rubber septum using a syringe.
4) After 2-3 minutes since the ISP injection, the reaction solution boiled, its temperature rising to 38 °C to 40 °C, and the reaction proceeded under refluxing at 35 °C to 36 °C; after 30 minutes to 40 minutes, once exhaustion of lithium was confirmed, LDEA synthesis was terminated.

### (2) Synthesis of 1-DCP lithium salt (Reaction Scheme 5)

1) The flask with the completed LDEA synthesis from the above process was left in a freezer and immersed in an ethanol bath cooled to -40 °C or less, or a refrigerant (-15 °C) containing ice and salt mixed in a ratio of 3:1 to cool the temperature of the solution to -10 °C or less.
2) Using a syringe, 50 mL of α-DIBCl (0.24 mol) was slowly injected into the flask through the rubber septum. Here, caution must be taken to ensure that the solution temperature does not exceed -10 °C while injecting α-DIBCl; this is because α-DIBCl reacts violently with LDEA, and therefore, α-DIBCl injected when the temperature of the solution is over -10 °C may cause the temperature of the solution to rise quickly and boil over.
3) Once the α-DIBCl injection was complete, the -40 °C ethanol bath was replaced with a 0 °C ice water bath. The temperature of the solution gradually rose in the ice water bath, and as the temperature of the solution reached 10 °C to 12 °C, formation of white precipitates (LiCI) began, forming a pure white suspension. Subsequently, the ice water bath was removed, and the temperature of the solution was raised to room temperature while stirring for 15 minutes to complete the synthesis of 1-DCP lithium salt.

### (3) Separation of 1-DCP lithium salt

1) Vacuum filtration: After placing and thus cooling the flask containing the reaction product of the white suspension in an ice water bath, the reaction product was vacuum-filtered to remove white precipitates (LiCI), and a clear yellow filtrate containing 1-DCP lithium salt was recovered. A glass frit funnel was used for vacuum filtration, and Celite 545 (Daejeong Chemicals) was used as a filter aid. Here, Celite must be dried at 120 °C for more than 2 hours before use; this is because if Celite contains moisture, a reaction product of 1-DCP lithium salt produced by reacting with moisture may clog the pores of the filter, making filtration difficult.
2) Vacuum evaporation: After removing the solvent and volatile reaction by-products from the yellow filtrate obtained following the vacuum filtration using a rotary evaporator, 1-DCP lithium salt was recovered (35.4 g, oily, brown). Here, the temperature of the water bath of the rotary evaporator was gradually raised to 50 °C from room temperature, and evaporation was conducted at 50 °C for 30 minutes. Here, if the water bath temperature is low or the evaporation time is short, reaction by-products may remain in the recovered 1-DCP lithium salt, which may cause a decrease in the purity of the final product (1-DCP).

### (4) Synthesis of 1-DCP from 1-DCP lithium salt (Reaction Scheme 6)

1) Neutralization of 1-DCP lithium salt: in a 250 mL beaker with about 100 mL of distilled water and ice placed therein, while maintaining the water temperature at 0 °C, while stirring using a stirring magnet, and by using a pipette, the synthesized 1-DCP lithium salt from the above process was slowly added. Here, because 1-DCP lithium salt tends to react violently with water, if the 1-DCP lithium salt is added too fast or the water temperature is too high during the above process, the temperature of the solution may rise rapidly and boil over, or even explode. Therefore, as the ice melted, additional ice was added to keep the solution cool, and the 1-DCP lithium salt was slowly added.
2) The solution was transferred to a 250-mL separatory funnel and the bottom layer (aqueous phase) was removed.
3) Washing of 1-DCP: the remaining liquid (supernatant, dark brown) in the separatory funnel was sequentially washed with about 100 mL of dilute hydrochloric acid (approximately 3 % HCl) and 100 mL of saturated salt water, and then, the crude 1-DCP in the supernatant was collected (25.4 g, brown). Here, the HCl washing was to remove residual amines, and the washing with salt water was to remove water.
4) Vacuum distillation: A vacuum distillation system was constructed by equipping a 50 mL two-necked round flask with a short path distillation head (cooling water temperature of -10 °C to 0 °C), a receiver (cooled in a ethanol water bath at - 40 °C to -20 °C), a 100 °C thermometer, a heating mantle, and a vacuum line. The crude 1-DCP (25.4 g) synthesized through the above process was added to the flask, and then distilled by heating under reduced pressure of 5 mBar to 10 mBar. The temperature of the solution decreased down to 5 °C-10 °C and then gradually increased; once the temperature of the solution reached 35 °C, the distillation was terminated, and 1-DCP was recovered (9 g of 1-DCP, colorless, d=0.77, bp =115 °C-120 °C, 95 % purity, 35 % yield). ¹H NMR (500 MHz, CDCl₃) δ 6.52-6.49 (m, 1H), 2.39 (d, J = 0.8 Hz, 2H), 0.99 (s,9H), 0.90 (d,J = 1.9Hz, 2H). ¹³C NMR (126 MHz, CDCl₃) δ 118.96, 99.06, 40.78, 30.03, 29.55, 5.91.

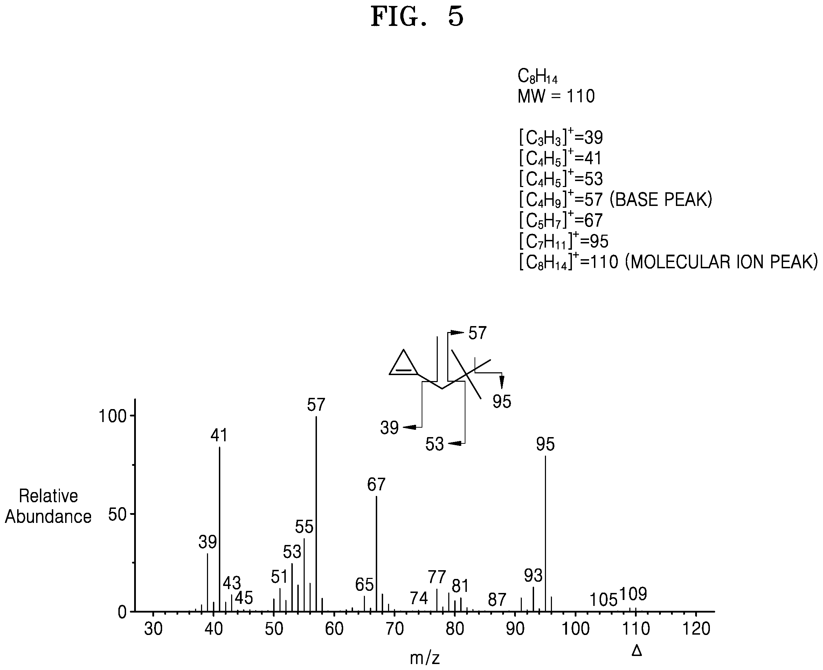

Mass spectrum analysis of 1-DCP showed a molecular ion peak ([C₈H₁₄]⁺=110) and a base peak ([C₄H₉]⁺= 57) (FIG. 5).

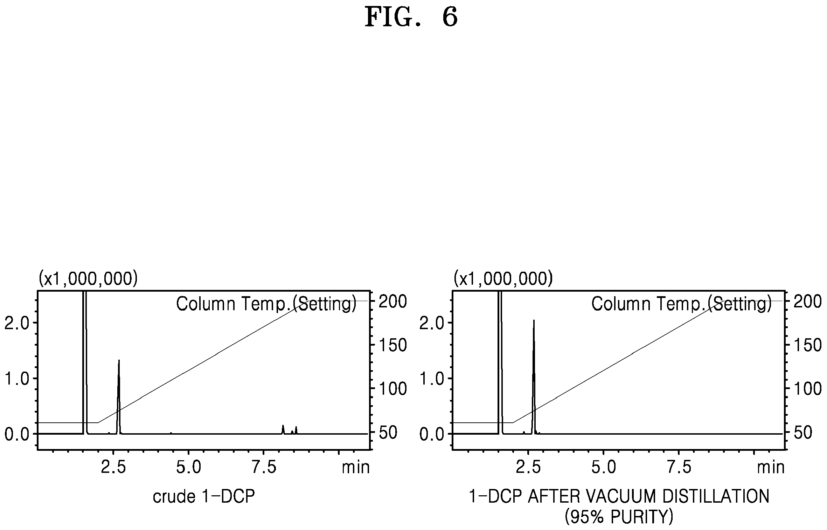

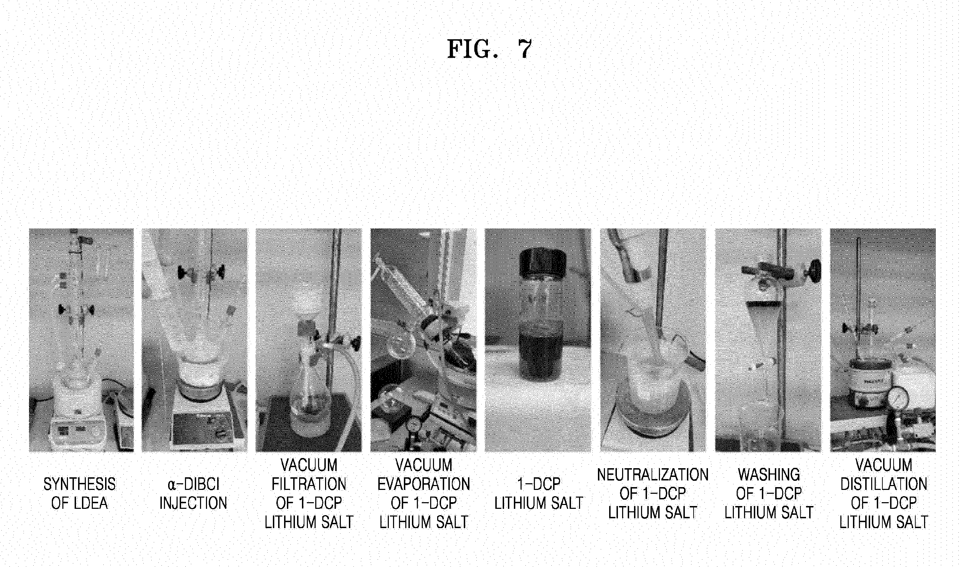

Meanwhile, the purity of the product of the above process was confirmed through gas chromatography (FIG. 6), and the synthesis process of 1-DCP is shown in FIG. 7.

### Example 2: Preparation of cyclodextrin inclusion complex of 1-DCP

The 1-DCP synthesized in Example 1 was able to be stored in a -40 °C freezer for several months or more; however, under refrigerated conditions, polymerization occurred within a few days, and storage at room temperature for more than one day was impossible. However, it was discovered that forming a complex by accommodating 1-DCP in cyclodextrin enables stable storage of 1-DCP at room temperature for several months or more.

### 2-1: Preparation of α-cyclodextrin inclusion complex

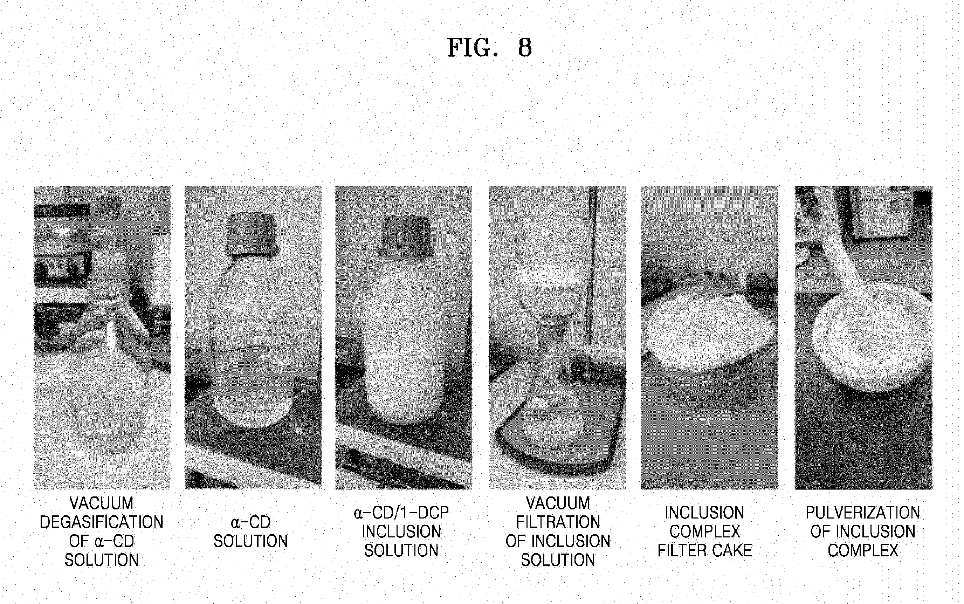

The method of preparing an inclusion complex of 1-DCP by using α-cyclodextrin, which relatively dissolves in water (solubility of 140 g/L) may be summarized as Reaction Scheme 8 below, the process of which is described in greater detail below. It should be noted that α-CD expressed in the following reaction scheme is shown as a schematic diagram, not a chemical formula. 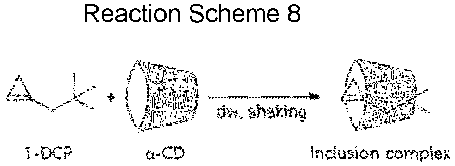
(1) Preparation of α-cyclodextrin solution: in a 1 L pressure-resistant bottle (maxP=1.5 bar) (Duran, Pressure plus bottle, code 10130780), 400 mL of distilled water and 40 g of α-CD (α-cyclodextrin, Henrikang Biotech) were added, and the bottle was closed with a stopper and was vigorously shaken to dissolve α-CD. The α-CD aqueous solution was degassed through repeated application and release of vacuum to the pressure bottle, 5 times.
(2) 5 mL of 1-DCP obtained in the above process was added to the α-CD aqueous solution and after closing the stopper, the solution was shaken vigorously for 15 minutes. Here, crystals of an inclusion complex of 1-DCP and α-CD were formed as white precipitates.
(3) The suspension containing the white precipitates was vacuum filtered using a Buchner funnel (Whatman filter paper, grade 3, particle retention 6 µm, thickness 390 µm), thereby recovering the white precipitates (inclusion complex crystals) as a filter cake. The recovered white precipitates were sealed in a desiccator and vacuum dried at room temperature for 24 hours to remove moisture, and then pulverized with a mortar and pestle, or an electric coffee grinder, to prepare a solid powder of the inclusion complex (1-DCP/α-CD molar ratio=0.73, 1-DCP content=92 µL/g). The process of preparing an α-cyclodextrin inclusion complex of 1-DCP is shown in FIG. 8.

### 2-2: Preparation of β-cyclodextrin inclusion complex

Cyclodextrins, which are synthesized from starch using enzymes, are produced in three types: alpha, beta, and gamma. Among these types, the beta type has the highest production rate, and therefore, the beta type is the cheapest of the three types. Therefore, in the preparation of an inclusion complex, the use of β-cyclodextrin may be economically advantageous. However, compared to the alpha type, the beta type has the disadvantage of having extremely low solubility in water.

However, it was discovered that inclusion of 1-DCP is possible when β-cyclodextrin is dissolved in dimethyl sulfoxide (DMSO) instead of water. The method by which an inclusion complex of 1-DCP was prepared by dissolving β-cyclodextrin in DMSO may be summarized in Reaction Scheme 9 below, and its detailed process was as follows. It should be noted that β-CD expressed in the following reaction scheme is shown as a schematic diagram, not a chemical formula. 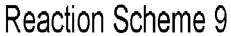 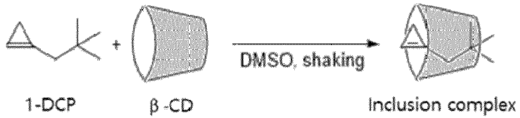
(1) Preparation of β-cyclodextrin solution: In a 40 mL vial, 10 g of β-CD (β-cyclodextrin hydrate, Samchun Chemicals) and 10 mL of dimethyl sulfoxide (DMSO) were placed, and mixed vigorously by a vortex mixer to dissolve β-CD.
(2) 1 mL of 1-DCP obtained in the above process was added to the β-CD solution and after closing the stopper, the solution was shaken vigorously for 15 minutes.
(3) When the solution was mixed with 100 mL of distilled water, a white suspension was formed, and by centrifugation of this suspension, white precipitates (inclusion complex) were recovered. The recovered white precipitates were vacuum-dried at 30 °C to remove moisture and were pulverized using a mortar and pestle to prepare solid powder (4 g) of 1-DCP/β-CD inclusion complex. However, the analysis of the amount of 1-DCP included in the inclusion complex showed that the inclusion efficiency was 17 µL/g, which was significantly lower than that of α-CD (92 µL/g).

### Example 3: Dissolution of 1-DCP inclusion complex and preparation of aqueous emulsion

To release 1-DCP from the α-CD inclusion complex of 1-DCP formulated in Example 2 to treat the same to a target plant, the ability to form an aqueous solution by dissolution in water is required. However, the cyclodextrin inclusion complex of 1-DCP, due to having very low solubility in water, could not be dissolved and prepared as a solution containing 1-DCP at a concentration suitable for plant treatment. However, it was discovered that 1-DCP/α-CD inclusion complex is readily dissolved in DMSO (dimethyl sulfoxide) at a rate of 4 g/10 mL, and it was possible to prepare an aqueous emulsion containing a suitable concentration of 1-DCP by having the 1-DCP inclusion complex first dissolved in DMSO and then mixed with water.

The method by which the 1-DCP inclusion complex was dissolved in DMSO and mixed with water may be summarized in Reaction Scheme 10 below, and the detailed process was as follows. It should be noted that the cyclodextrin expressed in the following reaction scheme is shown as a schematic diagram, not a chemical formula. 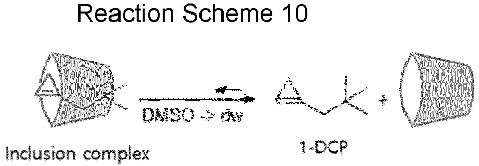
(1) Into a 20-mL vial, 4 g of 1-DCP/α-CD inclusion complex (containing 368 µL of 1-DCP) and 10 mL of DMSO were placed and then, the inclusion complex was dissolved through repeated ultrasonic dissolution and vigorous shaking for 2-3 minutes to thereby prepare 1-DCP/DMSO solution (12.7 mL).
2) The 1-DCP concentration of the 1-DCP/DMSO solution was 200 mM, and by mixing with water at a ratio of 400 times (0.5 mM) or 200 times (1 mM), a 1-DCP aqueous emulsion with a concentration of 0.5 mM to 1 mM, which is suitable for plant treatment, was prepared.

### Example 4: Effect of 1-DCP treatment on plants

The following experiments were conducted to evaluate the efficacy of 1-DCP synthesized in Examples above in inhibiting ethylene action.

### 4.1: Effect of 1-DCP soaking treatment on banana fruit

The following experiment was conducted to evaluate the effect of 1-DCP immersion treatment in ethylene inhibition.

In particular, immature banana fruits were harvested from a banana farm in Sancheong-gun, Gyeongsangnam-do and transported to the laboratory, and on the same day, the fruits were separated into two groups: the fruits (16) in Group A (control group) were immersed in a 0.025 % wetting agent (Carba, FarmHannong) solution not containing 1-DCP, and the fruits (16) in Group B (treatment group) were immersed in a 1 mM 1-DCP aqueous emulsion for 1 second, respectively, to wet the surface with the solution, and then dried at room temperature. Subsequently the next day, half of the fruits (8 each) from groups A and B were divided into two 40 L containers, sealed, and then injected with ethylene gas, thus exposing the fruits to 10 ppm of ethylene for 24 hours. The treated banana fruits were then stored at room temperature and observed for the degree of ripening after 7 days and after 14 days.

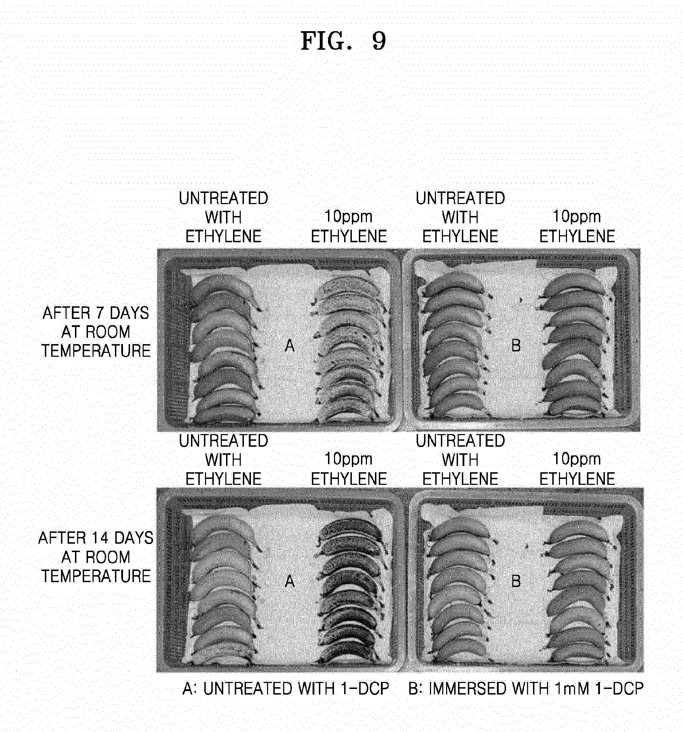

The result of the above experiment showed that the fruits in Group A exposed to ethylene turned yellow within 7 days and blackened within 14 days, whereas the 1-DCP immersion-treated fruits in Group B, despite being exposed to ethylene, maintained a more immature state than the fruits of Group A up to 14 days (FIG. 9).

Banana fruits are one of commonly known fruits that show accelerated maturation with high ethylene sensitivity. As a result of the above experiment, it was confirmed that an immersion treatment with 1-DCP at a concentration of 1 mM or less effectively inhibits ethylene action in banana fruits.

### 4.2: Effect of spray treatment of 1-DCP on Taechu sweet persimmon fruits

The following experiment was conducted to evaluate the effect of 1-DCP spraying treatment on ethylene inhibition.

In particular, four sweet persimmon trees of the Taechu variety were selected on October 7th at the Sweet Persimmon Research Institute Orchard located in Jinyeongeup, Gyeongsangnam-do, and the fruits growing on two of these trees (control group) were treated with a solution containing 0.025% wetting agent (Carba, Farmhannong), and the fruits of the other two trees (treatment group) were sprayed with an aqueous emulsion containing 1 mM 1-DCP and 0.025% wetting agent using a manual sprayer. Fruits in each treatment group were harvested on October 24, and measured/observed for changes in flesh hardness after 7 days and 14 days while being stored at room temperature.

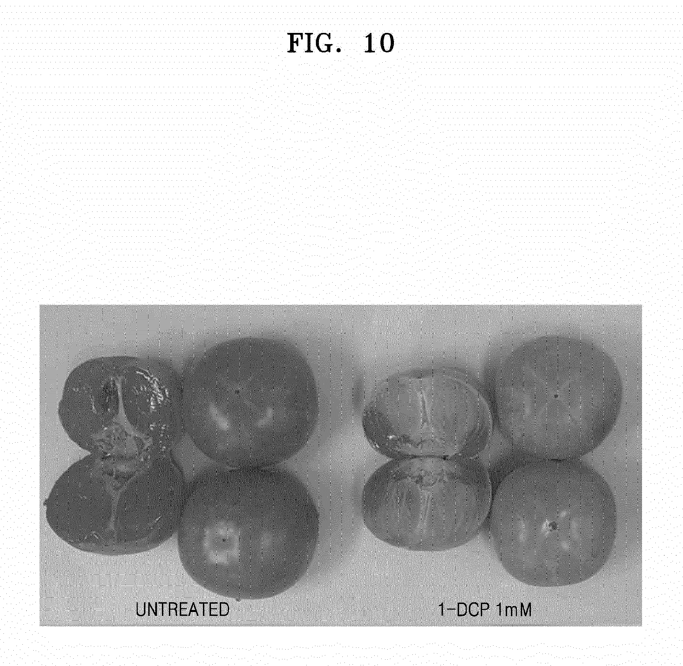

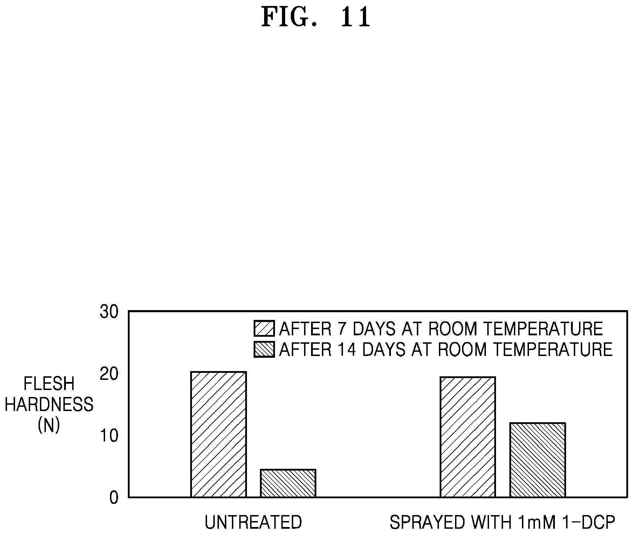

The above experiment showed that after 7 days of storage at room temperature, the flesh hardness of the fruits in the no-treatment group and the 1-DCP treatment group were measured to be 20.2 N and 19.5 N, respectively, showing no significant difference between the treatment groups. However, after 14 days of storage at room temperature, the flesh hardness in the control group and the treatment group was measured to be 4.3 N and 11.9 N, respectively; the fruits in the control group showed significant softening where their flesh became severely mushy, whereas the fruits in the 1-DCP spray-treatment group still retained their flesh in a relatively hard state (FIGS. 10 and 11).

It is generally known that the softening of fruit flesh in fruits is caused by the action of ethylene. As a result of the above experiment, it was confirmed that a spray treatment with 1-DCP at a concentration of 1 mM or less effectively inhibits ethylene action in the Taechu fruits.

The description of the present disclosure is for illustrative purposes, and those skilled in the art will understand that the present disclosure may be easily modified into other specific forms without changing the technical idea or essential features of the present disclosure. Therefore, the embodiments described herein should be understood in all respects as illustrative, and not restrictive.

The present disclosure provides a method of synthesizing 1-DCP [1-(2,2-dimethylpropyl)-cyclopropene], a compound that inhibits ethylene action in plants, and a method of utilizing the same. The method disclosed herein may allow the 1-DCP 1) to be stably synthesized and stored at room temperature, and 2) formed as an aqueous emulsion which can be used to immerse or be sprayed to a plant to protect the plant from ethylene action.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope of the disclosure as defined by the following claims.

## Claims

1. A method of preparing 1-(2,2-dimethylpropyl)-cyclopropene (1-DCP), the method comprising:
1) producing α-DIBCl [2-(chloromethyl)-4,4-dimethyl-1-pentene] by reacting α-DIB (α-diisobutylene) with hypochlorite and an acid;
2) producing a 1-DCP [1-(2,2-dimethylpropyl)-cyclopropene] alkali metal salt by reacting the α-DIBCl with an alkali metal alkylamide; and
3) producing 1-DCP by reacting the 1-DCP alkali metal salt with water or an alcohol,
wherein the acid comprises one or more selected from hydrochloric acid or a Lewis acid containing chlorine.

2. The method of claim 1, wherein the α-DIB is separated and obtained from an α/β-isomer mixture containing α-DIB and β-DIB.

3. The method of claim 2, wherein the separating and obtaining the α-DIB comprises:
a) producing β-DIBCI by treating an α/β-isomer mixture containing α-DIB and β-DIB with hypochlorite and an acid; and
b) separating α-DIB from a mixture containing the produced β-DIBCI and α-DIB.

4. The method of claim 1, wherein the Lewis acid containing chlorine comprises one or more selected from FeCl₃, AlCl₃, CeCl₃, and MoCl₅.

5. The method of claim 1, wherein the hypochlorite is selected from sodium hypochlorite (NaOCI), potassium hypochlorite (KOCI), lithium hypochlorite (LiOCI), and calcium hypochlorite [Ca(OCl)₂].

6. The method of claim 1, wherein the alkali metal alkylamide comprises one or more selected from lithium diethylamide, lithium diisopropylamide, sodium diethylamide, sodium diisopropylamide, potassium diethylamide, and potassium diisopropylamide.

7. The method of claim 1, wherein the 1-DCP alkali metal salt is one selected from a 1-DCP lithium salt, a 1-DCP sodium salt, and a 1-DCP potassium salt.

8. The method of claim 1, wherein the process 2) further comprises obtaining the produced 1-DCP alkali metal salt by separation.

9. A 1-DCP [1-(2,2-dimethylpropyl)-cyclopropene] compound represented by Formula 1:

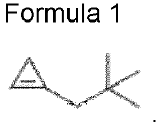

10. The compound of claim 9, wherein the compound is prepared by the method of claim 1.

11. A 1-(2,2-dimethylpropyl)-cyclopropene (1-DCP) inclusion complex, comprising 1-DCP and a cyclodextrin.

12. An ethylene response-inhibiting composition, comprising 1-(2,2-dimethylpropyl)-cyclopropene (1-DCP), a hydrate thereof, a solvate thereof, a salt thereof, or a complex thereof.

13. The ethylene response-inhibiting composition of claim 12, wherein the composition is for regulating, in a plant, one or more physiological activities selected from among germination and breaking dormancy of seeds, buds, tuberous roots, tubers, and bulbs, root growth and adventitious root formation, geotropism, stem elongation and thickening, flower blooming and development of female/male flowers, dropping of leaves, flowers, and fruits, respiration, ethylene production, stress tolerance, disease resistance or disease tolerance, formation or breakdown of pigments, fruit softening, and scent production.
